# EUROPÄISCHE PATENTSCHRIFT

(11) **EP 0 912 162 B1**
(45) Veröffentlichungstag und Bekanntmachung des Hinweises auf die Patenterteilung: **19.09.2001**
(21) Anmeldenummer: 97928247.2
(22) Anmeldetag: 19.06.1997
(51) Int. Cl.: A61K 7/16, A61K 9/00

(54) **FESTE ORALE, ANTIKARIOGENE ZUSAMMENSETZUNG ZUM REINIGEN DER MUNDHÖHLE UND ZÄHNE SOWIE VERFAHREN ZUR HERSTELLUNG DERSELBEN**
SOLID ORAL ANTICARIOGENIC COMPOSITION FOR CLEANING THE BUCCAL CAVITY AND TEETH, AND PROCESS FOR PREPARING THIS COMPOSITION
COMPOSITION ANTICARIEUSE ORALE SOLIDE POUR NETTOYER LA CAVITE BUCCALE ET LES DENTS, ET PROCEDE PERMETTANT DE LA PREPARER

(30) Priorität: 19.07.1996 DE 19629167
(43) Veröffentlichungstag der Anmeldung: 06.05.1999
(73) Patentinhaber: Einhorn Apotheke Dr. Günter Hanke, 74072 Heilbronn (DE)
(72) Erfinder: HANKE, Günther, D-74224 Flein (DE)
(74) Vertreter: Werner, Hans-Karsten, Dr.Dipl.-Chem.
(86) Internationale Anmeldenummer: EP9703204
(87) Internationale Veröffentlichungsnummer: WO9803151

(56) Entgegenhaltungen:
- EP-A- 0 354 442
- DE-A- 3 941 490
- US-A- 3 911 099
- US-A- 4 170 633
- US-A- 4 971 798

## Beschreibung

Gegenstand der vorliegenden Erfindung ist eine feste orale, antikariogene Zusammensetzung in Form einer Lutschtablette zum Reinigen der Mundhöhle und Zähne sowie ein Verfahren zur Herstellung dieser Zusammensetzung.

Es ist allgemein bekannt, daß nach dem Verzehr von Nahrungsmitteln - insbesondere von Süßwaren - durch Kohlenhydrate wie Zucker im Zahnbelag Säuren gebildet werden, die ihrerseits Zahnkaries verursachen.

Zucker, insbesondere weißer raffinierter Zucker (Saccharose), bewirken und fördern Karies, besonders wenn sie oft zwischen den Mahlzeiten verzehrt werden. Weiterhin ist bekannt, daß die durch Zucker im Zahnbelag gebildeten Säuren wie Ketosen, A1dosen, Hexosen etc. die Zahnoberfläche entmineralisieren und Kavitäten erzeugen. Im Zahnbelag entstehen die Säuren innerhalb weniger Minuten nach dem Verzehr von zuckerhaltigen Nahrungsmitteln. Innerhalb von fünf Minuten Einwirkung von Saccharose ist die Acidität des Zahnbelags hundertmal höher als der vor der Einwirkung registrierte Wert.

Aus dem Stand der Technik sind zum Neutralisieren von Zahnbelagssäuren alkalische Verbindungen wie zum Beispiel Natriumhydrogencarbonat, Natriumcarbonat, Ammoniumphosphat und andere bekannt.

Nachteil dieser alkalischen Puffer ist der salzige Geschmack, so daß dadurch die Akzeptanz beim Verbraucher stark verringert wird.

Ferner wurde von Clark, R., Hay, D.I. et al.: Brit. D. J. 111: 244 (1961) und Gilders, B.T.: Brit. D. J. 110: 17 (1961) der Gebrauch saurer "Zahnreinigungstabletten" empfohlen, die einen intensiven Fluß natürlichen Speichels anregen. Nachteil dieser Tablette war allerdings die mangelnde Neutralisierung des Zahnbelags.

Aus dem neueren Stand der Technik wird in der EP-B-0 486 563 eine Kaugummi-Zusammensetzung mit beschleunigter, kontrollierter Freisetzung aktiver Substanzen beschrieben. Die aktiven Substanzen umfassen diätetische Ergänzungsmittel, Antiseptika, Antirauchermittel, Süßstoffe, Aroma- und Heilmittel. Als Trägerstoff werden Harz-Komponenten wie zum Beispiel Terpen-Harz, Gummi-Collophonium oder Glycerin-Ester von teilweise hydriertem Holz eingesetzt. Als Lösungsvermittler dienen zum Beispiel Polyoxyethylen-Sorbit-Fettsäureester, Polyglycerinester und Sorbitester von Fettsäuren.

Die WO 92/02149 beschreibt kalorienreduzierte, feuchtigkeitsunempfindliche Kaumassen bestehend aus Polydextrose und Isomalt oder Manitol, sowie Verfahren zur Herstellung derselben.

Die DE-42 21 054 beschreibt ein Präparat zur prophylaktischen und therapeutischen Behandlung von Karies, dadurch gekennzeichnet, daß der Grundmasse aus Zucker oder Zuckeraustauschstoffen calcium- und phosphathaltige Substanzen zugesetzt werden, sowie ein Verfahren zur Herstellung dieses Präparates.

Die EP-B-0 222 846 beschreibt eine feste orale, antikariogene Zusammensetzung in Form eines Kaugummis oder eines Plätzchens zum Neutralisieren von Säuren in Zahnbelägen. Neben Sorbit als Trägerstoff und anderen herkömmlichen Inhaltsstoffen enthalten die Kaugummis und Plätzchen Harnstoff in einer Menge von 0,05 bis 80 Gew.-%. Da Sorbit eine leicht kariogene Wirkung besitzt, wird zusätzlich noch Harnstoff zugesetzt, von dem bekannt ist, daß er die Säurebildung hemmt.

Allerdings ist auch bekannt, daß das Säurebindevermögen von Harnstoff nicht sehr stark ausgeprägt ist. Ein weiterer Nachteil des Harnstoffes ist darin zu sehen, daß keine lebensmittelrechtliche Beurteilung vorliegt.

Die Erfindung hat sich somit die Aufgabe gestellt, eine feste orale, antikariogene Zusammensetzung zum Reinigen der Mundhöhle und Zähne zur Verfügung zu stellen, welche die oben genannten Nachteile nicht aufweist. Das Verfahren zur Herstellung der festen oralen, antikariogenen Zusammensetzung soll unter Verwendung an sich bekannter Mittel erfolgen und dabei einfach und kostengünstig durchführbar sein.

Die erfindungsgemäße Aufgabe wird überraschend einfach gelöst durch eine feste orale, antikariogene Zusammensetzung in Form einer Lutschtablette zum Reinigen der Mundhöhle und Zähne, die neben üblichen Hilfsstoffen, Aroma- und Süßstoffen 65 bis 95 Gew.-% Isomalt, 1 bis 25 Gew.-% Carbonate, 0,1 bis 5 Gew.-% Citrate und 0,1 bis 5 Gew.-% Phosphate enthält.

Als Trägerstoff wird zahnschonendes Isomalt (E 953, auch Palatinit® genannt) in einer Menge von vorzugsweise 80 bis 90 Gew.-% eingesetzt. Der Disaccharid-Alkohol Isomalt kann anstelle von Saccharose oder Zuckeraustauschstoffen in nahezu allen Lebensmitteln mit süßem Geschmack eingesetzt werden und hat darüber hinaus gegenüber Sorbit (E 420) den Vorteil, nicht kariogen und somit zahnschonend zu sein. Isomalt ist aufgrund der weitgehend insulinunabhängigen Metabolisierung für Diabetiker geeignet.

Als säurebindende Stoffe werden Carbonate, vorzugsweise 6 bis 10 Gew-% Natriumhydrogencarbonat, Citrate, vorzugsweise Calcium-und/oder Kaliumcitrat sowie Phosphate, vorzugsweise Calciumphosphat eingesetzt. Darüber hinaus haben diese Stoffe den Vorteil, daß sie lebensmittelrechtlich beurteilt werden. Gemäß Zusatzstoff-Zulassungs-Verordnung fallen diese Stoffe unter die allgemein zugelassenen Zusatzstoffe (s. Zusatzstoff-Verkehrs-VO, Liste 6 und Liste 10). Obwohl es sich bei der erfindungsgemäßen Lutschtablette um ein Kosmetikum handelt, werden die säurebindenden Stoffe im Gegensatz zu harnstoffhaltigen Präparaten (zum Beispiel gemäß EP-B-0 222 846) lebensmittelrechtlich beurteilt.

Die erfindungsgemäße basische Salzmischung aus Carbonaten, Citraten und Phosphaten neutralisiert die durch Speisen entstehenden Säuren und schützt damit die Zähne vor Karies.

Als Aromastoffe dienen beispielsweise Menthol und/oder Pfefferminz- und/oder Dentalaroma. Die Zahl der verwendbaren Aromastoffe ist praktisch unbegrenzt. Sie verleihen der Lutschtablette den frischen Geschmack.

Als Süßstoffe werden vorzugsweise Natriumcyclamat und/oder Saccharin eingesetzt.

Als Hilfsstoff bzw. Emulgator wird Magnesiumstearat eingesetzt.

Gegenüber Kaugummis, wie sie aus dem Stand der Technik bekannt sind, hat die erfindungsgemäße Lutschtablette den Vorteil, daß sie sich langsam im Mund auflöst, während ein zerkautes Kaugummi nach gewisser Zeit sein Aroma verliert und dann meistens ausgespuckt wird (Umweltbelastung!). Zusätzlich ist die Akzeptanz einer Lutschtablette gerade bei der älteren Generation wesentlich höher als die eines Kaugummis.

Die Herstellung der erfindungsgemäßen Lutschtablette ist relativ einfach und wird im pharmazeutischen Bereich mit der Bezeichnung "Direktverpressung" beschrieben.

In dem folgenden Beispiel wird die erfindungsgemäße Zusammensetzung und das Verfahren zu ihrer Herstellung näher erläutert:

### Beispiel

Das erfindungsgemäße Mengenverhältnis (bezogen auf 100 kg) der Zusammensetzung ergibt sich aus folgenden Bestandteilen:

| | |
|---|---|
| Isomalt (E 953) | 86,8 kg |
| Natriumhydrogencarbonat | 8,0 kg |
| Calciumcitrat | 0,67 kg |
| Kaliumcitrat | 0,67 kg |
| Calciumphosphat | 0,67 kg |
| Pfefferminzaroma, Menthol, Dentalaroma, Magnesium-stearat; Natriumcyclamat, Saccharin als Süßstoff | 3,19 kg |

Die oben genannten pulverförmigen Bestandteile werden in ihrer Beschaffenheit so ausgesucht, daß sie für eine Direktverpressung geeignet sind. Dies bedeutet in der Regel ein Korngrößenspektrum zwischen 100 und 400 *µ*m Durchmesser.

Das Menthol wird dann in dem Dentalaroma gelöst und am Ende der Mischung auf die Preßmasse gesprüht. In einem geeigneten Mischer werden Saccharin und Natriumcyclamat vorgemischt. Zu dieser Mischung wird Calciumphosphat hinzugegeben. Diese Mischung wird mit Calcium- und Kaliumcitrat vermischt. Danach werden Magnesiumstearat und das Pfefferminzaroma hinzugefügt, anschließend Natriumhydrogencarbonat und am Schluß anteilweise Isomalt.

Auf diese Mischung wird, wie vorher beschrieben, mit einem geeigneten Sprühsystem die Lösung des flüssigen Dentalaromas mit Menthol aufgegeben. Danach wird die Mischung in einem geeigneten, handelsüblichen Pulvermischer gemischt und sofort zur Direkttablettierung auf eine pharmaübliche Tablettenpresse (Rundläufer) gegeben und zu geeigneten Lutschtabletten verpreßt.
Die Härte der Lutschtabletten muß so beschaffen sein, daß diese das anschließende automatische Abfüllen in ein geeignetes Behältnis (zum Beispiel Blechdose) ohne Beschädigung überstehen.

Besonders wirksam ist die Lutschtablette, wenn man nach jeder Mahlzeit je nach Bedarf eine bis drei Lutschtabletten langsam im Mund zergehen läßt, am besten in der Backentasche.

## Patentansprüche

1. Feste orale, antikariogene Zusammensetzung zum Reinigen der Mundhöhle und Zähne, **dadurch gekennzeichnet, daß** sie außer üblichen Hilfsstoffen, Aroma- und Süßstoffen
65 bis 95 Gew.-% Isomalt
1 bis 25 Gew.-% Carbonate,
0,1 bis 5 Gew.-% Citrate,
0,1 bis 5 Gew.-% Phosphate
enthält.

2. Zusammensetzung nach Anspruch 1, **dadurch gekennzeichnet, daß** das Carbonat Natriumhydrogencarbonat ist.

3. Zusammensetzung nach Anspruch 1 oder 2, **dadurch gekennzeichnet, daß** als Citrate Calcium- und/oder Kaliumcitrate eingesetzt werden.

4. Zusammensetzung nach einem der Ansprüche 1 bis 3, **dadurch gekennzeichnet, daß** als Phosphat Calciumphosphat eingesetzt wird.

5. Zusammensetzung nach einem der Ansprüche 1 bis 4, **dadurch gekennzeichnet, daß** als Aromastoffe Menthol und/oder Pfefferminz- und/oder Dentalaroma eingesetzt werden.

6. Zusammensetzung nach einem der Ansprüche 1 bis 5, **dadurch gekennzeichnet, daß** als Süßstoff Natriumcyclamat und/oder Saccharin eingesetzt wird.

7. Zusammensetzung nach einem der Ansprüche 1 bis 6, **dadurch gekennzeichnet, daß** als Hilfsstoff Magnesiumstearat eingesetzt wird.

8. Zusammensetzung nach einem der Ansprüche 1 bis 7, **dadurch gekennzeichnet, daß** der Gehalt an Isomalt 80 bis 90 Gew.% beträgt.

9. Zusammensetzung nach einem der Ansprüche 1 bis 8, **dadurch gekennzeichnet, daß** der Gehalt an Carbonat 6 bis 10 Gew.-% beträgt.

10. Zusammensetzung nach einem der Ansprüche 1 bis 9, **dadurch gekennzeichnet, daß** sie in Form einer Lutschtablette vorliegt.

11. Verfahren zur Herstellung einer Zusammensetzung nach einem der Ansprüche 1 bis 10, **dadurch gekennzeichnet, daß** durch stufenweises Mischen der einzelnen Bestandteile in einem geeigneten Mischer ohne thermische Belastung oder Granulierhilfsmittel eine pressfertige Masse hergestellt wird.

## Claims

1. A solid oral anticariogenic composition for cleaning the oral cavity and the teeth, **characterized by** containing
from 65 to 95% by weight of isomalt;
from 1 to 25% by weight of carbonates;
from 0.1 to 5% by weight of citrates; and
from 0.1 to 5% by weight of phosphates,
in addition to conventional adjuvants, flavors and artificial sweeteners.

2. The composition according to claim 1, **characterized in that** said carbonate is sodium hydrogencarbonate.

3. The composition according to claim 1 or 2, **characterized in that** calcium and/or potassium citrates are employed as said citrates.

4. The composition according to any of claims 1 to 3, **characterized in that** calcium phosphate is employed as said phosphate.

5. The composition according to any of claims 1 to 4, **characterized in that** menthol and/or peppermint flavor and/or dental flavor are used as flavors.

6. The composition according to any of claims 1 to 5, **characterized in that** sodium cyclamate and/or saccharin is employed as an artificial sweetener.

7. The composition according to any of claims 1 to 6, **characterized in that** magnesium stearate is employed as an adjuvant.

8. The composition according to any of claims 1 to 7, **characterized in that** its content of isomalt is from 80 to 90% by weight.

9. The composition according to any of claims 1 to 8, **characterized in that** its content of carbonate is from 6 to 10% by weight.

10. The composition according to any of claims 1 to 9, **characterized by** being in the form of a sucking tablet.

11. A process for producing a composition according to any of claims 1 to 10, **characterized in that** a pressable mass is prepared by stepwise mixing of the individual ingredients in a suitable mixer without a thermal load and without using pelleting aids.

## Revendications

1. Composition anticarieuse orale solide pour nettoyer la cavité buccale et les dents, **caractérisée en ce qu'**elle contient, outre les adjuvants, aromatisants et édulcorants usuels
65 à 95 % en poids d'isomalt,
1 à 25 % en poids de carbonates,
0,1 à 5 % en poids de citrates,
0,1 à 5 % en poids de phosphates.

2. Composition selon la revendication 1, **caractérisée en ce que** le carbonate est l'hydrogénocarbonate de sodium.

3. Composition selon la revendication 1 ou 2, **caractérisée en ce qu'**on utilise en tant que citrates le citrate de calcium et/ou de potassium.

4. Composition selon l'une des revendications 1 à 3, **caractérisée en ce qu'**on utilise en tant que phosphate le phosphate de calcium.

5. composition selon l'une des revendications 1 à 4, **caractérisée en ce qu'**on utilise en tant qu'aromatisants du menthol et/ou de l'arôme de menthe poivrée et/ou un arôme dentaire.

6. Composition selon l'une des revendications 1 à 5, **caractérisée en ce qu'**on utilise en tant qu'édulcorant le cyclamate de sodium et/ou la saccharine.

7. Composition selon l'une des revendications 1 à 6, **caractérisée en ce qu'**on utilise en tant qu'adjuvant le stéarate de magnésium.

8. Composition selon l'une des revendications 1 à 7, **caractérisée en ce que** la teneur en isomalt est de 80 à 90 % en poids.

9. Composition selon l'une des revendications 1 à 8, **caractérisée en ce que** la teneur en carbonate est de 6 à 10 % en poids.

10. Composition selon l'une des revendications 1 à 9, **caractérisée en ce qu'**elle se présente sous forme d'une tablette à sucer.

11. Procédé de fabrication d'une composition selon l'une des revendications 1 à 10, **caractérisé en ce que**, par mélange pas à pas des différents constituants dans un mélangeur approprié, sans contrainte thermique ni auxiliaires de granulation, on prépare une masse prête à être moulée .
